# EUROPEAN PATENT SPECIFICATION

(11) **EP 0 381 427 B1**
(45) Date of publication and mention of the grant of the patent: **21.02.1996**
(21) Application number: 90300927.2
(22) Date of filing: 30.01.1990
(51) Int. Cl.: C12P 21/00, C12P 21/08, C12N 15/13, A61K 39/395, A61K 39/00, A61K 39/12, A61K 39/118, A61K 39/02, A61K 39/002, G01N 33/569

(54) **Vaccine Composition**
Vakzin-Zusammensetzung
Composition de vaccin

(30) Priority: 01.02.1989 AU 2504/89; 01.02.1989 AU 2505/89
(43) Date of publication of application: 08.08.1990
(73) Proprietor: THE UNIVERSITY OF MELBOURNE, Parkville, Victoria 3052 (AU); MEAT RESEARCH CORPORATION, Sydney, New South Wales 2000 (AU)
(72) Inventor: Meeusen, Elsa Nicole Theresia, Kensington, VIC 3550 (AU); Brandon, Malcolm Roy, Ivanhoe, VIC 3079 (AU); Gorrell, Mark Douglas, Annandale, NSW 2038 (AU); Walker, John, Balwyn, VIC 3103 (AU); Bowles, Vernon Morrison, North Glen Irish, VIC 3146 (AU)
(74) Representative: Harding, Richard Patrick

(56) References cited:
- EP-A- 286 405
- ULLMANNS ENCYKLOPÄDIE DER TECHNISCHEN CHEMIE, ed. 4, vol. 21, 1982, Verlag Chemie, Weinheim (DE); pp. 274-290/
- LEXIKON DER BIOLOGIE, vol. 4, 1988, Herder, Freiburg (DE); pp. 335-339/
- RÖMPPs CHEMIE LEXIKON, ed. 9, 1989, Georg Thieme Verlag, Stuttgart (DE); pp. 209-211, 1947-1953/

## Description

The present invention relates to antibody probes and the use of such probes in a process for detection and purification of a number of protective and diagnostic antigens, the preparation thereof and their use in the formation of vaccine compositions.

Considerable effort has been devoted in the prior art to the development of vaccines to control parasitic, bacterial and other infections of animals including livestock. However, little progress has been achieved to this end in the past 5 years although the associated technology of producing foreign products in large amounts in eukaryotic and prokaryotic organisms has advanced enormously. The identification of protective antigens in important pathogenic infections in animals, for example, has remained the principal stumbling block to the generation of vaccines.

One of the principal reasons for this is the enormous complexity of, for example, parasitic organisms which may have up to 10% of the genetic information of a mammal, and as a consequence have the ability to produce an enormous array of products at various stages of their life cycle, only a few of which may be important in developing an effective vaccine. In most instances, the researcher is confronted by hundreds of potential antigens. The central puzzle still remaining is which are the parasite antigens that elicit host-protective immune responses. The cloning of a parasite antigen chosen on the basis of - hope or inspiration, at considerable difficulty, expense and time, in theory may result in the development of an effective vaccine. However, in most instances, this approach has resulted in failure.

In the prior art in parasitic infections the emphasis has been placed on the screening of crude parasite antigen mixtures, parasite cDNA or genomic libraries with whole serum antibodies used as "probes". Serum contains large numbers of antibodies against other pathogens and antigens. In addition, most antibodies against the parasite are directed against non-protective antigens.

Very little effort has been made to improve the antibody probes used to screen crude parasitic antigen mixtures or parasite cDNA libraries, although this is vital for the detection of protective antigens and the monitoring of protective epitopes of these antigens during their subsequent molecular cloning.

For example, Haemonchus contortus is an intestinal parasite of sheep that localises in the abomasum (fourth stomach). Late larval and adult stages of the parasite feed on whole blood. The parasite is responsible for sizeable economic loss to the sheep industry in Australia and considerable loss overseas as it is a potentially fatal disease. Despite these losses no successful vaccine has been developed in the prior art against this parasite.

Caseous lymphadenitis (abbrev. CLA, also called Cheesy Gland) is a chronic infection of sheep and goats that is caused by the bacterium Corynebacterium pseudotuberculosis (syn. C.ovis). A complex cell-free vaccine for CLA (GLANVAC, Commonwealth Serum Laboratories) is known in the prior art and is currently administered either alone or as part of a 6 component antibacterial vaccine (6 in 1). The protection afforded by this vaccine is attributed to the inactivated toxin( i.e. toxoid) component. The toxin has a relative molecular weight of approximately 31 k daltons when run on 12.5% SDS-PAGE under reducing conditions. Whilst this prior art vaccine does generate some protective effect, the vaccine is complex and expensive, and significant numbers of infections may still occur.

Fasciola hepatica (liver fluke) is a parasitic infection which develops in the liver and bile ducts in sheep and cattle. Liver fluke may cause chronic and acute losses in the sheep and cattle industry. Numerous prophylactic and therapeutic treatments are known in the prior art but their effects have proved limited and liver fluke remains a chronic veterinary disease.

Taenia hydatigena is a parasitic infection which also develops in the liver of sheep. It is transferred from animal to animal by infected dogs and may generate some losses in the sheep industry. No successful vaccine has been developed in the prior art against this parasite.

It is accordingly an object of the present invention to overcome, or at least alleviate, one or more of the difficulties related to the prior art.

Accordingly, in a first aspect, the present invention provides a method for producing at least one antibody against a disease pathogen, said method including combining the steps of:
(a) providing a biological sample containing antibody-producing cells from an immune animal infected with, or challenged by, a pathogen or a pathogen extract, said biological sample being taken within a suitable period from an infection site, or an area of a lesion, or an area close to or draining the infection site or lesion;
(b) isolating the antibody-producing cells from the biological sample;
(c) culturing the antibody-producing cells from the biological sample in vitro in a suitable culture medium; and
(d) harvesting the antibody produced by the antibody-producing cells.

The invention also provides an antibody prepared by such a method, and an antibody probe including at least one such antibody against a disease pathogen.

The animal from which the biological sample is taken may be of any suitable type; and the biological sample is advantageously taken within the period that the pathogen is vulnerable to immune attack after the animal has been challenged by the pathogen or pathogen extract. The animal may be a mammal including humans. The mammal may be a domestic animal such as sheep or cattle.

In the following description, reference will be made to a method of identifying parasitic and bacterial immunogens important in diseases of sheep and cattle more specifically t the parasitic infections Taenia hydatigena (cestode) , Haemonchus contortus (namatode), Fasciola hepatica (trematode) and the bacterial pathogen Corynebacterium pseudotuberculosis. It should be understood, however, that such immunogens and pathogens are illustrative only and the method is generally applicable to animals including humans. In particular, the method described herein may be used to detect the immunogen in auto-immune diseases.

The biological animal sample may be of any suitable type. The biological sample may be from animal tissues, organs, blood, lymph or lymph nodes. The biological sample may be taken from any section of the infected animal. However, it is preferred that the samples be taken from the infected site or an area of a lesion which may be formed in certain diseases or an area close to the infected site or a lesion such as in the lymph nodes.

However, serum/plasma samples are not preferred as the biological samples according to this aspect of the present invention. It has been found that the majority of antibodies found in a serum/plasma sample are irrelevant to protection or specific diagnosis of a pathogen or are unrelated to the pathogen. In addition, other serum/plasma components may interfere with the specific reactions between pathogen components and antibodies to them.

In contrast, the probes described in the present invention are highly enriched in pathogen-specific antibodies and can be selected to be restricted to the pathogen-stage of particular importance to protective immunity.

It is preferred that the biological samples are taken from the animals at a predetermined time in the development of a disease. In general, for a parasitic infection, it has been found that the biological samples should be taken a short time after an infection with a pathogen or after injection with products obtained from a pathogen. It is postulated that a parasite is vulnerable for only a short time after entering the subject after which it changes structure and is no longer vulnerable to immune attack and may no longer induce protective antibodies.

The cells isolated from the biological sample may include B cells. The cells may be isolated similarly at a time known to include a secretion and/or antibody producing period. Alternatively, the cells may include memory cells which may be generated at a later stage in certain diseases.

Thus, preferably the cells are taken a short time after in vivo stimulation, preferably within approximately 2 to 13 days thereafter with, for example, the relevant parasite stage thereby resulting in the in vivo induction of antibody forming cells which will secrete specific antibodies into the culture medium after in vitro incubation. No, or very few antibodies may be secreted in culture medium without prior in vivo stimulation of resting lymphocytes.

In vitro secretion of antibodies in the culture medium by recently activated B cells may be enhanced by the addition of helper factors to the cultures. The helper factors may be cytokines used alone or in combination, including Interleukin 1, 2, 3, 4, 5, 6, 7 and 8, colony stimulating factors, interferons and any other factors that may be shown to have an enhancing effect on specific B cell secretion.

The method of producing an antibody according to this aspect of the present invention may include a further step of activating the cells isolated to proliferate and secrete and/or release antibodies.

The cell activation step may include adding a cell activating agent to the culture medium. The cell activating agent may be parasite-derived or may be selected from mitogens and helper factors produced by leucocytes, or their synthetic equivalents or combinations thereof.

The mitogens may be selected from products derived from pokeweed (Phytolacca americana) also known as pokeweed mitogen (PWM), polyvinylpyrrolidone (PVP), polyadenylic-polyuridylic acid (poly(A-U)), purified protein derivate (PPD), polyinosinic-polycytidilic acid (poly(I-C)), lipopolysaccharide (LPS), staphylococcal organisms or products thereof, Bacto-streptolysin O reagent (SLO), Staphylococcal phage lysate (SPL), Epstein-Barr virus (EBV), Nocardia water-soluble mitogen (NWEM), phytohemagglutinin (PHA) Concanavalin A (Con A) and dextran-sulphate and mixtures thereof. The cell proliferation agent may be any agent that indirectly or directly results in B cell proliferation and/or antibody secretion such as solid-phase anti-immunoglobulin. The helper factors may be cytokines including interleukin 1, 2, 3, 4, 5, 6, 7 and 8, colony stimulating factors, interferons and any other helper factors that may be shown when added alone, or in combination with other factors and agents to have an enhancing effect on specific B cell proliferation and/or antibody secretion. This in no way is meant to be an exhaustive list of mitogens and cell actuating agents including helper factors.

The in vitro culturing of the cells may be conducted with or without prior steps to separate sub-populations of cells. The harvesting of antibodies may be conducted by harvesting of the supernatant from the culture medium. This supernatant contains antibodies secreted by these cells during the in vitro culture or artificially released from the B cells, for example by lysis of the B cells. It has been found, surprisingly, that the antibody-containing supernatants may be used directly to detect antigens of a pathogen.

Accordingly, in a second aspect of the present invention, there is provided a method of preparing an antigen associated with a disease pathogen, said method including:
(a) providing a sample of a pathogen taken from a diseased animal at a stage of development during which it is thought to be most susceptible to attack;
(b) providing an antibody probe according to the first aspect of the invention;
(c) probing the pathogen sample with the antibody probe to detect at least one antigen recognised by the antibody; and
(d) isolating the antigen detected.

In a third aspect of the present invention there is provided a method of preparing a protective antigen that provides protection for an animal against a disease pathogen, said method including the steps of:
(a) providing a sample of the pathogen;
(b) providing an antibody probe according to the first aspect of the invention including at least one antibody against an antigen associated with the said pathogen.
(c) probing the pathogen sample with the antibody probe to detect at least one protective antigen; and
(d) isolating the antigen which provides protection for an animal against the pathogen.

The disease pathogen may be of any suitable type. The disease pathogen may be derived from any infectious agents including viruses, chlamydias, rickettsias, mycoplasmas, bacteria, spirochetes, fungi, protozoa, helminths (trematodes, nematodes, cestodes) and ectoparasitic arthropodes (eg. ticks, mites, blowflies) or may be an autoantigen or tumour antigen.

The disease pathogen is preferably a parasite, parasite extract or parasitic section thereof. The disease pathogen may be selected from Haemonchus contortus, and intestinal parasite of sheep, Fasciola hepatica (liver fluke) a parasitic infection which develops in the liver and bile ducts in sheep and cattle, or Taenia hydatigena a parasitic infection which also develops in the liver of sheep. Other parasites include Lucilia cuprina, Trichostrongylus spp, Boophilus spp, Ostertagia spp, Schistosome spp, Taenia spp and Echinococcus spp. However, the invention is not restricted thereto and the description following is merely illustrated by reference to these parasites.

In an alternative aspect, the pathogen sample may be taken from a bacterium. For example, the bacterium Corynebacterium pseudotuberculosis may be used.

The disease pathogen sample is taken at a stage of development of the pathogen during which it is thought to be most susceptible to attack. For example, for a parasitic cestode infection, it may be suitable to take the sample from the oncosphere stage. It has been found that antigens present in the oncosphere stage of a parasitic infection are not present, for example, in the metacestode stage. For a parasitic worm infection, it may be suitable to take the sample from the larval, preferably late larval stage. For a parasitic fluke infection, it may be suitable to take the sample from the juvenile fluke stage.

The sample of disease pathogen may be mixed with a standard buffer solution and placed on a standard support such as an SDS-polyacrylamide gel to separate the proteins contained therein. The separated proteins may then be transferred to nitro-cellulose, nylon or other sheets.

The probing with a suitable antibody may further include subjecting the product produced thereby to a detection assay. The detection assay may include western blot techniques. The detection assay may be an immunoprecipitation assay, a radioimmunoassay, an enzyme-linked immunoassay or immunofluorescent assay.

The at least one antibody produced as described above may be utilised simply in the form of the supernatant harvested from the culture medium. Alternatively, the antibodies may be separated and purified.

The antigen located as described above may be detected utilising any suitable assay technique.

In a further preferred aspect of the present invention the antibody contained in the culture medium may be used for the affinity purification, preferably immunoaffinity purification of antigen.

Accordingly in a preferred aspect there is provided a method of purifying an antigen, said method including:
(a) providing a crude antigen mixture;
(b) providing an antibody against a disease pathogen, said antibody being immobilised on a suitable support and having been produced by a method according to the first aspect of the invention;
(c) subjecting the crude antigen mixture to affinity chromatography utilising the immobilised antibody; and
(d) isolating the purified antigen of the pathogen recognised by the immobilised antibody.

Antibody can be obtained from the culture supernatant probe by conventional methods. For example methods usually used to purify immunoglobulins from serum or plasma, eg. precipitation with ammonium sulphate, fractionation with caprylic acid, ion exchange chromatography or by binding and elution from immobilised protein G or protein A may be utilised. Antibody so obtained can then be coupled to suitable supports, eg. CNBr-activated Sepharose 4B (Pharmacia) Affi-gel (Bio-RAD) or other affinity chromatography supports able to bind proteins.("Sepharose" is a Registered Trade Mark).

Immobilised antibody can then be applied to the fractionation and purification of specific antigen from a complex parasite extract by affinity chromatography. After binding of antigen to immobilised antibody, unbound macromolecular species can be washed away from the solid support with, eg buffers containing 1.5M NaCl. Subsequently the antigen can be eluted from the affinity column with, eg low or high pH buffer or buffers containing chaotropic ions, eg 0.5 - 3.0 M sodium thiocyanate.

The application of the antibody probe to affinity chromatography enables sufficient quantities of specific antigens to be rapidly isolated from a complex crude extraction mixture for biochemical characterisation, amino-acid sequencing and vaccination of animals for limited protection studies. Application of affinity chromatograpny for obtaining antigen(s) avoids the difficulties often encountered when applying conventional biochemical techniques to the purification of an antigen about which little or not data is known. It also obviates the need to raise polyclonal or monoclonal antibodies for the purpose of "analytical" affinity chromatography. Large scale preparation may however, require the preparation of polyclonal or monoclonal antibodies.

The antigens isolated or located may be used in the preparation of monoclonal antibodies. The monoclonal antibodies may form the basis of a passive treatment of the disease discussed above. Having identified the antigen(s) molecular biology or chemical techniques, eg cloning techniques may be used to produce unlimited amounts of this antigen or alternatively synthetic peptides corresponding to different fragments of the identified antigens may be used as a means to produce a vaccine.

Accordingly, in a preferred aspect of the present invention, there is provided a method of preparing a synthetic antigenic polypeptide that provides protection for an animal against a disease pathogen, which method includes:
(a) preparing a cDNA library, or genomic library derived from a sample of a disease pathogen;
(b) providing an antibody probe according to the first aspect of the invention;
(c) generating synthetic polypeptides from the cDNA library or genomic library;
(d) probing the synthetic polypeptides with the antibody probe; and
(e) isolating the synthetic antigenic polypeptide detected thereby.

Either cDNA or genomic libraries may be used. The cDNA or genomic libraries may be assembled into suitable expression vectors that will enable transcription and the subsequent expression of the clone DNA, either in prokaryotic hosts (eg. bacteria) or eukaryotic hosts (eg. mammalian cells). The probes may preferably be selected from:
(i) synthetic oligonucleotide probes based on the amino acid sequence of the antigen identified and purified as described above,
(ii) antibodies obtained from the culture medium produced as described above,
(iii)monoclonal or polyclonal antibodies produced against the antigens identified and purified as described above,
(iv) recombinant or synthetic monoclonal antibodies or polypeptides with specificity for the antigen, eg. as described by Ward et al 1989, Nature 241, pages 544-546.

The protective antigens may function as diagnosticantigens as discussed below.

Accordingly, in a preferred aspect of the present invention, there is provided a protective antigen against Taenia hydatigena infections, selected from antigens having approximate molecular weights of 25 and 34 kilodaltons, as hereinafter described. As cross protection between various cestodes has been documented, similar antigens may also be detected in other cestode species, eg. T. saginata , T. ovis, T. solium, Echinococcus granulosus.

In a further preferred aspect of the present invention there is provided a protective antigen against Haemonchus contortus infections, having an approximate molecular weight of 67 to 75 kilodaltons, as hereinafter described.

In a further preferred aspect of the present invention there is provided a protective antigen against Fasciola hepatica infections, having an approximate molecular weight of 120 to 125 kilodaltons, as hereinafter described.

In a still further preferred aspect of the present invention there is provided a protective antigen against Corynebacterium pseudotuberculosis infections, having an approximate molecular weight of 38 to 40 kilodaltons, as hereinafter described. This antigen is a protein antigen.

Accordingly, in a further aspect of the present invention, there is provided a method of producing a monoclonal antibody against an antigen of a disease pathogen, which method includes:
(a) providing a B cell capable of producing antibodies against said antigen and obtained from an animal immunised with a protective antigen against the disease pathogen prepared by the method according to the third aspect of the invention;
(b) fusing the B cell with the myeloma cell;
(c) propagating a hybridoma formed thereby; and
(d) harvesting the antibody produced by said hybridoma.

The present invention further provides a vaccine or veterinary composition including a prophylactically effective amount of at least one protective antigen prepared by the method of the third aspect against a disease pathogen selected from Taenia hydatigena, Haemonchus contortus, Fasciola hepatica or Corynebacterium pseudotuberculosis as hereinafter described.

The present invention further provides a vaccine or veterinary composition including a therapeutically effective amount of at least one monoclonal antibody prepared as described above.

The vaccine or veterinary composition according to the present invention may be administered orally or may be administered parenterally (for example by intramuscular, subcutaneous or intravenous injection). The amount required will vary with the antigenicity of the active ingredient and need only be an amount sufficient to induce an immune response typical of existing vaccines.

Reactive experimentation will easily establish the required amount. Typical initial doses of vaccine or veterinary compositions may be approximately 0.001-1 mg active ingredient/kg body weight. The dose rate may increase or multiple doses may be used as needed to provide the desired level of protection.

The vaccine or veterinary composition according to the present invention may further include a veterinary acceptable carrier, diluent or excipient therefor. Preferably the active ingredient may be suspended or dissolved in a carrier. The carrier may be any solid or solvent that is non-toxic to the animal and compatible with the active ingredient. Suitable carriers include liquid carriers, such as normal saline and other non-toxic salts at or near physiological concentrations, and solid carriers, such as talc or sucrose. Adjuvants, such as Freund's adjuvant, complete or incomplete, or immunomodulators such as cytokines may be added to enhance the antigenicity of the antigen if desired. When used for administering via the broncial tubes, the vaccine is suitably present in the form of an aerosol.

The present invention will now be more fully described with reference to the following examples. It should be understood, however, that the description following is illustrative only and should not be taken in any way as a restriction on the generality of the invention described above.

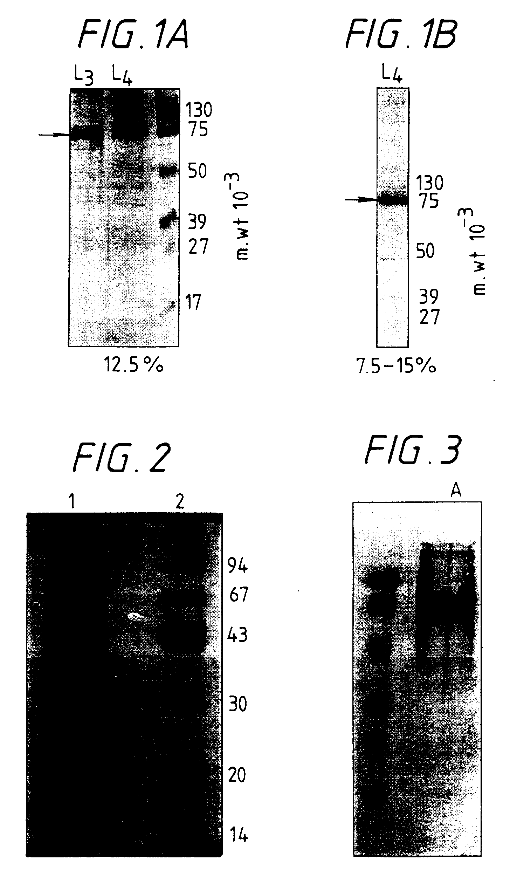

In the figures:
FIGURE 1A - H. contortus
   Western blot from a 12.5% SDS polyacrylamide gel showing antigens in the L₃ and L₄ preparation (arrowed) identified by culture supernatant from immune-challenged sheep. Prestained molecular weight standards (BIORAD) are indicated.
FIGURE 1B - H. contortus
   Western blot from a 7.5 to 15% gradient SDS-polyacrylamide gel showing antigens in the L₄ preparation (arrowed) identified by culture supernatant from immune-challenged sheep. Prestained molecular weights (Bio-Rad) are indicated.
FIGURE 2 - H. contortus
   Silver-stained gel of affinity purified proteins on a 12.5% SDS-polyacrylamide gel under non-reducing conditions. The affinity-purified proteins are in lane 1 and molecular weight standards (Pharmacia) are given (mw x 10³). The region of antibody reactivity is bracketed.
FIGURE 3 - H. contortus
   Western blot of affinity isolated antigens after probing with culture supernatant from a immune-challenged animal (Lane A). Prestained molecular weight markers (Bio-Rad) as in Figure 1.
FIGURE 4 - H. contortus
   Western blot of affinity isolated antigen after incubating with Proteinase K (lanes A), trypsin (lanes B), Glycopeptidase F (lanes C) and control (no enzyme) (Lanes D). Molecular weights (Pharmacia) (mw x 10³) are indicated.
FIGURE 5 - H. contortus
   IEF capillary transfer to nitrocellulose from an agarose IEF gel ampholyte range 3 - 5 and probed with culture supernatant from an immune animal. Ph range using IEF standards (Bio-Rad) are given.
FIGURE 6 - F. hepatica
   Western blot from a 7.5 - 15% SDS-PAGE gel.
   MW = prestained molecular weight markers (BIORAD) as in Figure 1.
   Lanes 1 & 4 - NEJ antigen preparation.
   Lanes 2 & 5 - Adult fluke antigen preparation.
   Lanes 3 & 6 - 12 day old fluke antigen preparation.
   Lanes 1, 2 & 3 were probed with culture supernatant from HLN cells of infected cattle.
   Lanes 4, 5 & 6 were probed with a mixture of culture supernatants from HLN of 10 day challenged sheep with an abreviated or chronic primary infection.
   Brackets = antigen claimed.

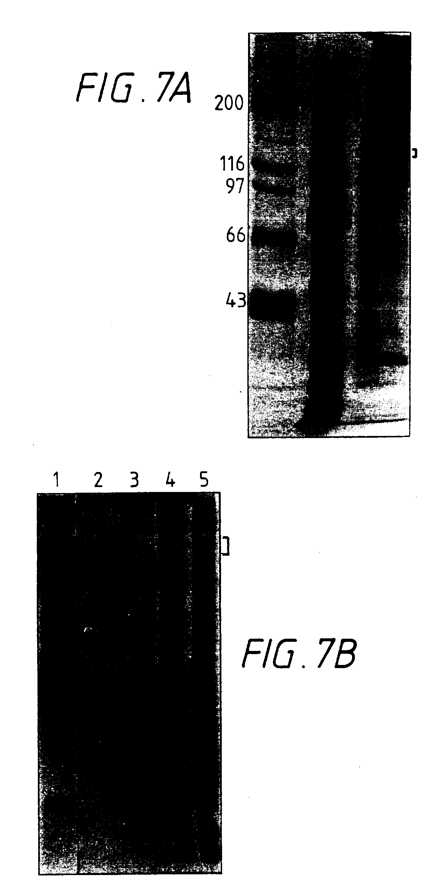

FIGURE 7 - F. hepatica
   (A) 7.5 - 15% SDS-PAGE gel stained with silver nitrate.
      - lane 1 -: high molecular weight markers (BIORAD) in reducing sample buffer.
      - lane 2 -: prestained molecular weight markers (BIORAD).
      - lane 3 -: Newly excysted juvenile (NEJ) antigen preparation in non-reducing sample buffer.
      - Brackets =: position of antigen claimed.
   (B) 10% SDS-PAGE gel stained with silver nitrate.
      - lane 1:: normal mouse serum in non-reducing buffer.
      - lane 2 & 3:: n-butanol extracted antigen preparation in non-reducing sample buffer. Pellet (2) and supernatant (3) after 100.000 g spin for 50 minutes.
      - lane 4 & 5:: Supernatant from NEJ sonicate. Pellet (4) and supernatant (5) after 100.000 g spin for 50 minutes.
      - Brackets =: antigen claimed.

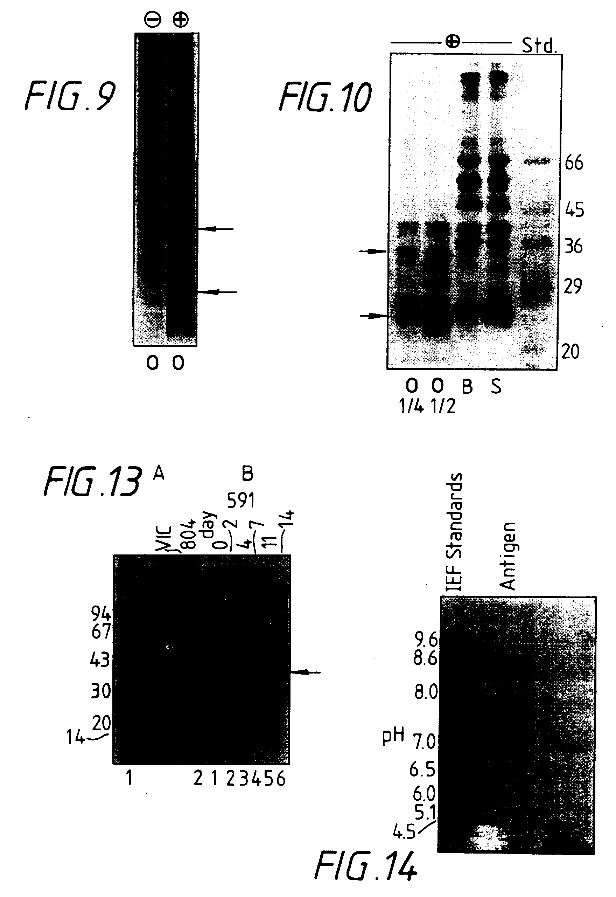

FIGURE 8 - F. hepatica
   10% SDS gel stained with silver nitrate.
   Bound (lane 2) and non-bound (lane 3) fraction after affinity purification of sonicated NEJ antigen preparation.
   Brackets = position of antigen claimed.
   Lane 1 = prestained molecular weight markers (BIORAD) as in Figure 1.
FIGURE 9 - T.hydatigena
   Western blot from a 10% SDS-PAGE gel of oncosphere antigen in non-reducing sample buffer probed with culture supernatant from liver lymphocytes isolated from immune-challenged ( ⊕ ) or non-challenged ( ⊖ ) sheep.
FIGURE 10 - T.hydatigena
   Probing of Western blots of oncosphere antigen (O) at dilutions of 1/2 and 1/4 and probing of Western blots of bladderwall metacestode antigen (B) preparations and scolex metacestode antigen (S) preparations with the positive culture supernatant as in Figure 9. 10% SDS-PAGE gel.
   Arrows = antigens claimed
   Sigma molecular weight markers run in reducing buffer.

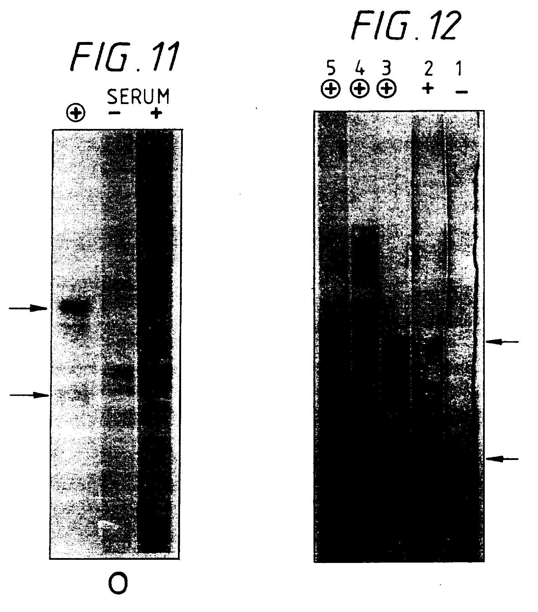

FIGURE 11 - T.hydatigena
   Probing of Western blots of oncosphere antigen with negative serum ( - ) or positive serum ( + ) taken from the same sheep at the same time after infection as the positive culture supernatant ( ⊕ ). 12% SDS-PAGE gel. Serum dilution: 1/20.
FIGURE 12 - T.hydatigena
   Western blot of oncosphere antigens probed with negative (1) or positive (2) serum or culture supernatant from leucocytes isolated from liver (3), hepatic lymph node (4) or prescapular lymph node (5) of recently infected animals. 10% SDS-PAGE gel.
FIGURES 13A AND 13B
   Western blot on nitrocellulose from 12.5% SDS-PAGE-Samples solubilised in reducing buffer.
   - 13A:: Stained with amido black.
   Lane 1. Molecular weight standards.
   Lane 2. WA 1030 isolate cell extract.
   - 13B:: WA 1030 isolate cell extract probed with sera at 1:100 dilution obtained from a sheep infected with C. pseudotuberculosis.
   Lane 1. Serum taken immediately prior to infection.
   Lane 2. 2 days past infection
   Lane 3. 4 days past infection
   Lane 4. 7 days past infection
   Lane 5. 11 days past infection
   Lane 6. 14 days past infection
   Arrows = protein antigen claimed.
FIGURE 14 - C. pseudotuberculosis

Isoelectric focusing of C. pseudotuberculosis antigen. Antigen has pH between 6.8 - 6.9.

### EXAMPLE 1

### Haemonchus contortus

Haemonchus contortus is an intestinal parasite of sheep that localises in the abomasum (fourth stomach). Late larval and adult stages of the parasite feed on whole blood. The parasite is responsible for sizeable economic loss to the sheep industry in Australia and considerable loss overseas and is a potentially fatal disease. Despite these losses no successful vaccine has been developed in the prior art against this parasite.

### Parasite and experimental animals

H. contortus third stage larvae (L₃) were collected from faecal cultures of donor sheep experimentally infected with the parasite. Immune animals were obtained by repeatedly infecting sheep with H. contortus larvae and then monitoring faecal egg output. When a challenge dose produced no eggs in the faeces the animal was said to be immune. Once immune, the sheep were left for a period of at least four wseks before being challenged with 50,000 - 200,000 L₃ larvae and then killed five days post challenge.

### Preparation of culture supernatants

Abomasal lymph nodes, draining the area of infection (abomasum) were removed and cell suspensions prepared as described for T. hydatigena below. Bulk cultures of 10 - 50 ml were set-up in culture flasks (Miles ) at concentration of 2 - 4 x 10⁶ cells/ml in culture medium. Preliminary experiments established that most of the antibodies in the culture supernatant were produced by the antibody secreting cells present in the in vivo stimulated lymph nodes and that this was not further increased by stimulation with pokeweed mitogen (PWM). PWM was therefore deleted from the cultures and culture supernatants were harvested after a five day incubation of cells at 37°C in a 5% CO₂ atmosphere and stored at -20°C until used.

### Stage-specific recognition of antigens by culture supernatant

Third stage larvae of H. contortus were exsheathed in 0.05% sodium hypochlorite for 10 minutes at 37°C to remove the second stage sheath. The larvae were then repeatedly washed and centrifuged at 3,000 g for 10 minutes in phosphate buffered saline (PBS) pH 7.4. After the sixth wash they were transferred to 500 ml of DME medium pH 6.8 in the presence of 200 U/ml penicillin and 0.2 ug/ml streptomycin and cultured at 39°C with 20% CO₂ in air for 5 days. The culture media was then centrifuged at 3,000 g for 15 minutes at 20°C. L₃, in vitro switched L₄ and adult parasites were removed and mechanically homogenised using a teflon pestle and a ground-glass tube for 20 minutes at 4°C in the presence of 0.1% Empigen™ zwitterionic detergent (Calbiochem) in PBS and 5mM phenylmethyl sulfonyl fluoride (PMSF). The homogenised larvae were immediately aliquoted and stored at -70°C. Frozen aliquots were thawed and mixed 1:1 with SDS non-reducing sample buffer and boiled for 5 minutes before being centrifuged for 2 minutes at 5,000 g and run on a 12.5% SDS-polyacrylamide mini gel (Bio-Rad) according to the method of Laemmli (1970). The gel was then electrophoretically transferred to nitrocellulose (Kerlero de Rosbo et al, 1984) and probed with serum or culture supernatants from abomasal lymph node cells from immune challenged or non-challenged immune sheep. Western blots were blocked with 0.5% Tween 20 in PBS and all washes were done in 0.05% Tween 20 in PBS. The second antibody was a rabbit anti-sheep immunoglobulin coupled to horse-radish peroxidase (Dako) and then developed with 3′3-diaminobenzidene (Sigma) and hydrogen peroxide.

Larval antigens were detected between approximately 67-75 kilodaltons (Kd) in the in vitro cultured fourth stage larvae (L₄), and third stage (L₃) exsheathed larvae of H. contortus when probed with culture supernatant from immune-challenged sheep (Figure 1). No antigens were identified at the molecular weights mentioned above for adult Haemonchus preparations (data not shown). There was no reaction when supernatant from non-challenged immune sheep was used to probe the blots (not shown), indicating that all the antibodies produced in the culture supernatant of the immune-challenged sheep were induced by the 5 day in vivo challenge. Similar probing of the Western blots with serum of the same animals taken at the same time reacted with several brands in all 3 parasite stages but did not highlight the 67 - 75 kD antigen (not shown). Also, as opposed to culture supernatant, no difference could be detected between serum of immune-challenged or immune non-challenged sheep.

### Preparation of L₄ antigen for affinity purification

Crude antigen for affinity purification was prepared by shearing in vitro switched L₄ larvae with a polytron tissue homogeniser for 30 seconds on ice in the presence of 0.05% Empigen in 0.1 M Tris pH 8.0 and 5mM PMSF. After polytron treatment, SDS was added to a final concentration of 2% and the sample boiled for 3 minutes in a water bath, then centrifuged at 35,000 rpm for 30 minutes at 4°C. After centrifugation the SDS was removed from supernatant by precipitating the protein according to "Method A" described by Henderson, Oroszlan and Konigsberg (1979). After SDS removal, 7M Guanidine hydrochloride (IBI) was added to the protein precipitate and allowed to stand for 5 hours on ice. An equal volume of 25% glycerol in distilled water was added and the entire sample dialysed against PBS and 0.05% Empigen pH 7.4. The sample was then applied to an affinity column.

### Affinity purification of antigen

An affinity column was constructed to isolate the specific antigen by firstly removing antibodies from the culture supernatant of immune-challenged sheep using both Protein G Sepharose (Pharmacia/LKB) and donkey anti-sheep antibodies (Dako) coupled to Affi-prep (Bio-Rad) according to manufacturers specifications. The purified antibodies were then coupled to Affi-prep (Bio-Rad) according to the manufacturer's instructions and the column equilibrated in PBS and 0.05% Empigen detergent.

L₄ antigens were loaded onto the affinity column and unbound proteins removed using 0.5M NaCl pH 7.4 and 0.05% empigen. Bound proteins were eluted using 1M NH₄SCN (Ajax) in PBS. The eluate was monitored by OD₂₈₀. After elution the sample was extensively dialysed against 0.005M Tris pH 8.3, lyophilised and stored at -70°C. Samples were resuspended in distilled water and used for further analysis.

### Silver-staining and Coomassie staining of affinity isolated antigen

On conventional SDS-Page gels several bands could be seen when silver staining the gels according to the method of Morrissey (1981) Figure 2. Immunoblotting and probing with culture supernatants from immune-challenged sheep resulted in a very intense antibody response in the 67- 75 kd region in the affinity isolated preparation (Figure 3). However no bands on either silver or coomassie stained gels could be positively correlated with this region of antibody reactivity. It would therefore appear that this particular molecule does not stain with the conventionally used protein stains.

### Enzyme digests

6 ug of affinity isolated antigen from fourth stage larvae was incubated overnight at 37°C with either Glycopeptidase F (20 ul) (Boehringer Mannheim) or proteinase K (10 ug) (Sigma) in 0.1M Na-phosphate buffer- pH 8.0 containing 10 mM EDTA, 0.1% SDS, 0.5% Triton™-X-100 and 0.1% β-mercaptoethanol. In addition the antigen was incubated with trypsin (10 ug) (Difco ) in PBS. After incubation, all samples were mixed with SDS non-reducing buffer, boiled for 5 minutes and loaded onto a 12.5% SDS-polyacrylamide gel, transferred to nitrocellulose and probed with culture supernatant as previously described. Both proteinase K and trypsin treatment resulted in breakdown of the protein such that there was no recognition by antibodies in the culture supernatant (lanes A and B respectively). Glycopeptidase F had no effect on the antigen producing the same result as the control incubation (lanes C and D). This indicates that the antigen has a protein component which is degradable by proteinase K and trypsin, however the protein does not appear to contain asparagine-linked glycans under these conditions.

### Iso-electric point of the antigen

Thin-layer iso-electric focussing (IEF) gels were prepared using a plastic template (Corning Immunoelectrophoresis plate) according to the method of McLachlan and Cornell (1978). Each IEF gel consisted of 0.95% w/v of IEF agarose (Bio-Rad), 11.4% w/v D-sorbitol (Sigma), 4.8% carrier ampholytes (Bio-Rad) range 3 - 5 or 3 - 10 and distilled water. The water, sorbitol and agarose were boiled, then placed on a 56°C water bath. The ampholines were then added, the solution poured onto the template and a piece of Gelbond™ (FMC Pharmacia) overlayed. The template was placed in a plastic bag and stored at 4°C for at least 2 hours before use.

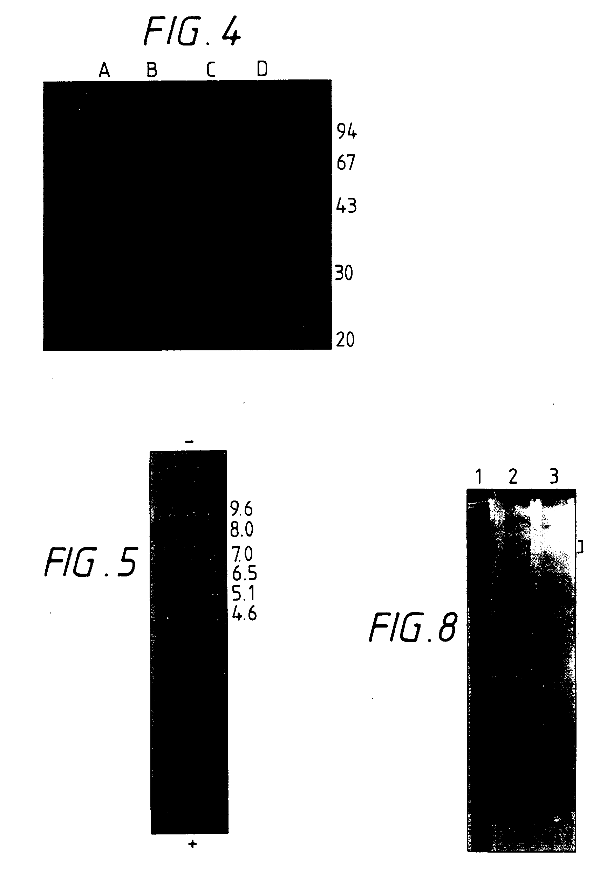

One ul of affinity purified antigen was applied and the gel was run at one watt constant power for 45 minutes. Upon completion of the run the gel was overlayed with nitrocellulose (Schleicher and Schuell) followed by several pieces of filter paper and a glass plate to act as a weight. Proteins then diffused from the gel to the membrane for one hour after which the membrane was blocked and probed with culture supernatant as previously described. The results indicated the presence of a highly acidic protein with an iso-electric point below 4.65 as indicated by IEF standards (Bio-Rad) Figure 5.

### EXAMPLE II

### Fasciola hepatica

Fasciola hepatica (liver fluke) is a parasite from the trematode family which can develop in the liver and bile ducts of many mammals and is of particular economic importance to the sheep and cattle industry. No prophylactic treatments such as vaccines against liver fluke are on the market. While sheep do not develop an immunity against F. hepatica, cattle can become partially resistant against reinfection. Although the mechanism of resistance in cattle has not been fully elucidated one possibility is that cattle recognise a different (protective) antigen than sheep. The technique described above was therefore used to study the differential antigen recognition between sheep and cattle.

### Antigen Preparations

### 1. Newly excysted juveniles (NEJ)

F.hepatica metacercariae (Mc) were purchased from Baldwin Aquatics (Monmouth, Oregon, U.S.A.). Metacercariae were excysted by suspension in 20 ml DME medium containing 100 mg Na-Tauro-cholic acid, 80 mg L-cysteine. The suspension was gassed in a mixture of 40% CO₂, 10% O₂ and 50% N₂, incubated at 37°C and NEJ collected after filtration through metal mesh. Sedimented NEJ were resuspended in phosphate buffered saline containing protease inhibitors (PBS-PI) (5mM Iodoacetamide and 1mM PMSF) and sonicated. Aliquots were stored at -70°C.

### 2. Juvenile Flukes

Mice were infected orally with 30 Mc and killed 12 days later. Macerated livers were washed through a tea strainer and the juvenile flukes were recovered by differential centrifugation. Antigen was prepared as above.

### 3. Adult Fluke

Infected livers from sheep or cattle were obtained from an abattoir. Adult flukes were recovered from the bile ducts, homogenised with a tissue grinder, aliquoted and frozen at -70°C.

### SDS-polyacrylamide gel electrophoresis (SDS-PAGE) and Western blotting

As for Haemonchus but only 7.5 - 15% gradient gels or 10% SDS-Page gels were run. All antigens were mixed 1:1 with non-reducing buffer. Bovine and ovine antibodies were detected using peroxidase-conjugated rabbit anti-bovine or sheep immunoglobulins (DAKO) respectively.

### Butanol extraction

Newly excysted juveniles suspended in PBS-PI were sonicated and spun for 10 minutes at 5000 g. The supernatant was used for affinity purification as described later. The pellet was resuspended in PBS-PI and an equal volume of cold n-butanol was added. The mixture was incubated on ice for 10 minutes with occasional vortexing and then spun at 5000 g for 5 minutes. The water-soluble fraction was collected in the bottom layer.

### Affinity purification

Antibodies were purified from the culture supernatant of infected cattle (see later) using sepharose bound protein G (Pharmacia) and used for the preparation of an affinity column essentially as described for H. contortus.

Triton X-100 R-S (Sigma) was added to a NEJ sonicate supernatant (see above) at a final concentration of 2%. The sonicate was loaded onto the affinity column and unbound fractions washed through with PBS containing 0.1% Triton X-100 R-S and 1 mM PMSF followed by 1.5 M NaCl. The bound fraction was eluted with 2 M NH₄SCN in PBS, dialysed against distilled water and freeze dried. Freeze-dried fractions were resuspended in PBS for further use.

### Preparation of culture supernatant

Three sheep and three cattle were infected orally with 400 metacercariae. 18 days later they were dosed with FASINEX 120 (CIBA-GEIGY Australia Ltd.) to eliminate the primary infection. The animals were left for an additional 35 days before being challenged orally with 400 metacercariae. They were killed 10 days after challenge. Hepatic lymph nodes (HLN) were removed, cells isolated and incubated at 2 - 4 X 10⁶ cells/ml in culture medium with PWM as described for T. hydatigena and the supernatants harvested after 7 days in vitro culture. Additional culture supernatant was collected from HLN cells of sheep carrying an untreated primary F. hepatica infection (80 Mc) and challenged with 300 Mc 10 days before slaughter.

### Results

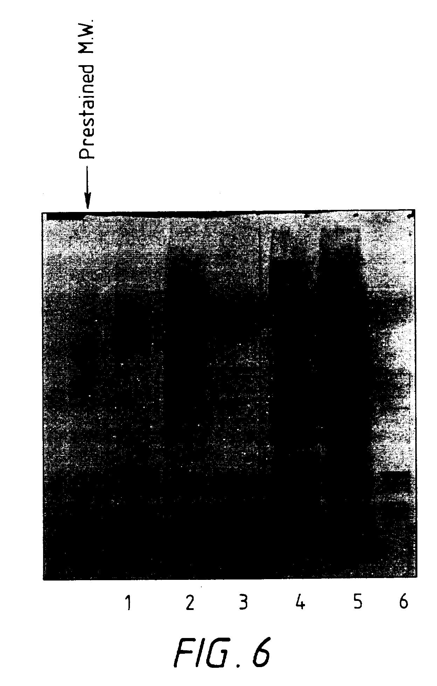

Probing of Western blots with culture supernatant from HLN cells of infected cattle resulted in strong antibody reactivity to a doublet antigen in the NEJ antigen preparation located slightly above the highest prestained molecular weight marker (Figure 6). Only one band at the higher molecular weight level of the NEJ doublet was consistently present in 12 day old fluke preparations while a similar band in the adult fluke preparation could not be reliably detected in several repeat experiments. In contrast,

when the antigen preparations were probed with a mixture of this sheep culture supernatant and culture supernatant from chronically infected and challenged sheep, no reaction was observed with the band recognised by cattle supernatant although other bands were strongly recognised (Figure 6).

Unlike the restricted reactivity of the culture supernatant, similar Western blots probed with serum taken from the same animals at the same time reacted much more diffusely with a number of bands in all 3 parasite stages and no obvious difference between cattle and sheep serum could be observed (not shown).

Silver staining of the total NEJ preparation did not clearly stain the antigen recognised by the cattle culture supernatant suggesting it is a minor component of the total molecular make-up of the parasite (Figure 7a). By measuring the position of the antigen on a replicate Western blot relative to the highest prestained molecular weight marker, its approximate molecular weight on SDS-PAGE gel using BIORAD high MW markers as standards was calculated at 120 - 125 Kd. Clear silver staining of the doublet in the NEJ preparation could be seen when a n-butanol extracted soluble fraction of a NEJ sonicate pellet was run on an SDS-PAGE gel (Figure 7b).

Affinity purification on the bovine antibody column resulted in a clear depletion of the antigen from the non-bound fraction and high enrichment in the bound fraction (Figure 8). In addition there was also a strong low molecular weight (±24 Kd) band in the bound fraction which also reacted strongly with the cattle culture supernatant on a Western blot (not shown) suggesting that the low MW band was due to breakdown of the 120 - 125 Kd antigen during the affinity procedure.

### EXAMPLE 3

### Taenia Hydatigena

### MATERIALS AND METHODS

### Parasite and experimental animals

T.hydatigena eggs were collected from mature worm segments after purging of infected dogs with arecoline hydrobromide. 2 year old sheep kept on farms were used. At this age sheep have generally acquired immunity to T.hydatigena through natural exposure and this was confirmed in preliminary experiments. Positive sera and positive culture supernatants were collected from sheep killed 13 days after intraruminal injection of 40-50,000 T.hydatigena eggs. Negative culture supernatants were collected from sheep without prior challenge infection. Negative serum was collected from 5 month old sheep reared under wormfree conditions.

### Preparation of leucocyte suspensions

Liver leucocytes were recovered by the following procedure. The sheep liver was removed and perfused via the portal vein with 1 litre of phosphate buffered saline (PBS) at room temperature followed by 0.5 liter of cold PBS with continuous and gentle massaging of the liver. This procedure resulted in complete blanching of the entire liver. Approximately 100 g of liver tissue was homogenized in a foodprocessor (Goldair, Australia) at low speed for 8-10 sec. The homogenized liver was then pushed through metal mesh, left to sediment for 8-10 min and filtered through cotton gauze. The cells were washed twice by centrifugation at 400 g for 8 min, followed by a low spin at 10 g for 5 min. to remove small clumps and the majority of hepatocytes. The supernatant of the last spin was collected and the remaining hepatocytes and dead cells removed by centrifugation over Ficoll/Isopaque gradients.

Leucocytes were also recovered from lymph nodes by cutting and teasing the nodes over a fine wire mesh. Dead cells and clumps were removed by centrifugation over Ficoll.

### Preparation of culture supernatant

Leucocytes were resuspended at a concentration of 2-4 x 10⁶/ml in culture medium consisting of DME to which was added 10 mM HEPES, 100u/ml penicillin, 100 ug/ml streptomycin, 2.5 x 10⁻⁵ M 2-mercaptoethanol, 2 mM glutamine, 1 mM pyruvate and 10% fetal calf serum. Two ml cultures were set up in 24 well Linbro™ plates, stimulated with 25 ug/ml of pokeweed mitogen (PWM, Gibco Labs., Grand Island, NY) and incubated at 37°C in a 5% CO₂ atmosphere. After 7 days the cultures were pooled and the cells sedimented by centrifugation. The supernatant was stored at -20°C until used.

### Preparation of antigens

(a) Oncosphere antigen (O)
   T.hydatigena eggs recovered from mature worm segments were hatched with sodium hypochlorite and the released oncospheres were purified over 100% Percoll. A total of 2.3 x 10⁵ oncospheres were resuspended in 1 ml PBS containing protease inhibitors (PBS-PI) (5 mM Iodoacetimide and 1 mM phenyl methyl sulphanyl-fluoride) and frozen at -20°C. This preparation was later thawed, sonicated with a MSE 150W ultrasonic disintegrator (Crawley, England), aliquoted and stored at -20°C.
(b) Metacestode antigens
   Metacestodes were collected from the peritoneal cavity of sheep at slaughter. The cyst fluid was removed, scolex and bladderwalls separated and frozen in PBS-PI at -20°C . The preparations were later thawed, homogenised in a Kinematica Homogeniser™ (Polytron, Luzern, Switzerland), sonicated and centrifuged at 1400 rpm x 15 min. The supernatants were aliquoted and frozen at -20°C.

### Detection of antigens by the Western blotting technique

40 ul antigen fractions were mixed with 40 ul SDS non-reducing sample buffer, boiled for 5 min. centrifuged for 10 min at 5000 g and run on a 10 or 12% SDS-polyacrylamide gel. The separated proteins were transferred to nitrocellulose sheets overnight. The sheets were blocked with 3% chicken ovalbumin (OVA) and cut into strips. Specific antigens on the strips were revealed after the following incubation steps:
(1) culture supernatant or serum,
(2) biotinylated donkey anti-sheep Ig (Amersham)
(3) streptavidin-biotinylated horseradish peroxidase complex (Amersham)
(4) peroxidase substrate (0.6 mg/ml diaminobenzidine in PBS containing 0.05% H₂O₂).
Reagents were used at manufacturers recommended dilutions.
(1) Probing of Western blots of oncosphere antigen preparations revealed that there were 2 distinct antigen bands specifically recognised by culture supernatant collected from liver leucocytes of recently infected sheep (⊕) that were not recognised by culture supernatant collected from liver leucocytes of unchallenged sheep (⊖) (Figure 9, arrows).
   These 2 antigen bands were still detected by the positive culture supernatant when the oncosphere preparation was diluted 1/2 and 1/4 and had approximate molecular weights of 22K and 35K (Figure 10).
(2) When using the same positive liver culture supernatant to probe the Western blots of bladderwall (B) or scolex (S) preparations, the 2 oncosphere antigen bands were not detected (Figure 10). This indicates that the 2 antigen bands specifically detected in the oncosphere preparation are stage specific for the oncosphere, the parasite stage most likely to be susceptible to immune attack.
(3) The 2 antigen bands detected when positive liver culture supernatant was used to "probe" the oncosphere Western blots were not detected when serum taken from the same sheep at the same time after infection was used as a probe (Figure 11).
(4) The 2 oncospheral antigen bands were also detected when using culture supernatant from leucocytes isolated from the lymph nodes of recently infected animals (Figure 12).

### EXAMPLE 4

### Corynebacterium pseudotuberculosis

Caseous lymphadenitis (abbrev. CLA, also called Cheesy Gland) is a chronic infection of sheep and goats that is caused by the bacterium Corynebacterium pseudotuberculosis (syn. C. ovis). A complex cell-free vaccine for CLA (GLANVAC™, Commonwealth Serum Laboratories) is known in the prior art and is currently administered either alone or as part of a 6 component antibacterial vaccine (6 in 1). The protection afforded by this vaccine is attributed to the inactivated toxin (i.e. toxoid) component. The toxin has a relative molecular weight of approximately 31 k daltons when run on 12.5% SDS-PAGE under reducing conditions. Whilst this prior art vaccine does generate some protective effect, the vaccine is complex and expensive, and significant numbers of infections may still occur.

This example describes the isolation of a protective antigen from Corynebacterium pseudotuberculosis infections, being approximate molecular weight of 38 - 40 kilodaltons.

### Identification of Antigen

Culture supernatants were obtained using essentially the same methods as described for Haemonchus contortus (Example 1). Challenge infections of immune and naive sheep were localised to the lower leg by injection of a suspension of viable C.pseudotuberculosis and the popliteal lymph node was taken and set up in culture 5 to 7 days later.

Western blot analysis of whole cells using culture supernatants showed:
(i) immune sheep recognised a complex pattern of antigens, but in the majority of animals the 38 - 40 kilodalton antigen was immunodominant
(ii) naive (non-immune) animals strongly recognised the 38 - 40 kilodalton antigen and usually showed little other reactivity.

### Extraction of antigen

Confluent growth of C.pseudotuberculosis is obtained on Brain Hearth Infusion Agar by aerobic incubation at 37°C for between 1 and 3 days. Cells are obtained by scraping from the solid medium.

The bacterial mass is resuspended in sterile water and washed by vortexing, then centrifugated at 3000 Xg for 15 mins. The supernatant is decanted and the wet cell pellet extracted by resuspension in approximately 4 times its volume of a solution of 1.0% (w/v) sodium dodecyl sulphate (SDS) in water and heating to between 70°C - 100°C for 10 minutes. Cells and debris are removed by centrifugation at 10,000 Xg. for 15 minutes. The crude mixture contains antigens other than that claimed.

The protein antigen can be purified from the complex mixture extracted with SDS from whole cells of C. pseudotuberculosis, as described above, or from cell-free culture supernatant. Conventional biochemical techniques can be used including ion exchange and molecular-sieve chromatography, ammonium sulphate fractionation together with hydrophobic chromatography. In addition, affinity chromatography can be used employing antibody isolated from the probe, immobilised on a suitable solid phase. Alternatively, polyclonal or monoclonal antibodies can be raised by immunisation of animals with purified antigen.

The antigen remains soluble in ammonium sulphate solutions up to 50% saturation.

### Characterisation of 38 - 40 kD antigen

The protein has an apparent molecular weight of between 38-40 kilodaltons when run under reducing conditions on a 12.5% SDS-polyacrylamide gel. It can be stained with Coomassie brilliant blue R250 and by silver stain. An amido black stain on nitrocellulose after Western blotting may also be used. Results are illustrated in Figure 13A.

Thin layer isoelectric focusing in 0.95% agarose containing 11.4% sorbitol and pH 5-8 ampholines localises the antigen to a pH 6.8-6.9 using the following Biorad IEF markers (see Figure 14): phycocyanin - 4.65, B-lactoglobulin B-5.10, bovine carbonic anhydrase 6.0, human carbonic anhydrase 6.5, equine myoglobin 7.0, human hemoglobin A 7.1, human hemoglobin C 7.5, cytochrome C 9.6.

Amino-acid sequence analysis of the native antigen that had been eluted from a SDS-polyacrylamide gel reveals a portion of the sequence at or near the amino terminus to be (progressing toward the carboxy terminus):

E S A T L S K E P L K A S P G R A D T, V G V Q (or if preferred Glu, Ser, Ala, Thr, Teu, Ser, Lys, Glu, Pro, Leu, Lys, Ala, Ser, Pro, Gly, Arg, Ala, Asp, Thr, Val, Gly, Val, Gln.

### References

- Henderson, L.E., Oroszlan, S. and Konigsberg, W. 1979. Analytical Biochemistry 93: 153 - 157.
- Kerlero de Rosbo, N., Carnegie, P.R., Bernard, C.C.A. and Linthicum, D.S. 1984. Neurochemistry Research, 9: 1359 -1369.
- Laemmli, U.K. 1970. Nature (London) 277: 680 - 685.
- McLachlan, R., and Cornell, F.N. 1978. Pathology, 10: 395.
- Morrissey, J.H. 1981. Analytical Biochemistry, 117: 307 - 310.
- Ward, E.S., Gussow, D., Griffiths, A.D., Jones, P.T. and Winter, G. 1989, Nature 341: 544 - 546.

## Claims

1. A method for producing at least one antibody against a disease pathogen, said method including combining the steps of:
(a) providing a biological sample containing antibody-producing cells from an immune animal infected with, or challenged by, a pathogen or a pathogen extract, said biological sample being taken within a suitable period from an infection site, or an area of a lesion, or an area close to or draining the infection site or lesion;
(b) isolating the antibody-producing cells from the biological sample;
(c) culturing the antibody-producing cells from the biological sample in vitro in a suitable culture medium; and
(d) harvesting the antibody produced by the antibody-producing cells.

2. A method according to claim 1, wherein the biological sample is taken from an animal within the period that the pathogen is vulnerable to immune attack after the animal has been challenged by the pathogen or pathogen extract.

3. A method according to claim 1 or 2, wherein the antibody-producing cells isolated from the biological sample are B cells isolated within a period known to include a secretion and/or an antibody-producing period.

4. A method according to claim 3, wherein the B cells are isolated within a period that the pathogen is vulnerable to immune attack after the animal has been challenged by the pathogen or pathogen extract.

5. A method according to any one of claims 1 to 4, wherein the antibody-producing cells isolated from the biological sample include memory B cells.

6. A method according to any one of claims 1 to 5, further including the step of adding a cell activating agent to the culture medium to activate and/or proliferate the cultured antibody-producing cells isolated from the biological sample to secrete and/or release antibodies.

7. A method according to claim 6, wherein the cell activating agent is selected from mitogens and helper factors or their synthetic equivalents, pathogen derived molecules or a combination thereof.

8. An antibody prepared by the method according to any one of claims 1 to 7.

9. An antibody probe including at least one antibody against a disease pathogen, said antibody having been produced by a method according to any one of claims 1 to 7.

10. A method of identifying antigens, said method including:
(a) providing a crude antigen mixture;
(b) providing at least one antibody against a disease pathogen, said antibody having been produced by a method according to any one of claims 1 to 7; and
(c) probing the crude antigen mixture with the antibody to identify antigens of the pathogen recognised by the antibody against the pathogen.

11. A method of purifying an antigen, said method including:
(a) providing a crude antigen mixture;
(b) providing an antibody against a disease pathogen, said antibody being immobilized on a suitable support and having been produced by a method according to any one of claims 1 to 7;
(c) subjecting the crude antigen mixture to affinity chromatography utilizing the immobilized antibody; and
(d) isolating the purified antigen of the pathogen recognized by the immobilized antibody.

12. A method of preparing an antigen associated with a disease pathogen, said method including:
(a) providing a sample of a pathogen taken from a diseased animal at a stage of development during which it is thought to be most susceptible to attack;
(b) providing an antibody probe according to claim 9;
(c) probing the pathogen sample with the antibody probe to detect at least one antigen recognised by the antibody; and
(d) isolating the antigen detected.

13. A method of preparing a protective antigen that provides protection for an animal against a disease pathogen, said method including the steps of:
(a) providing a sample of the pathogen;
(b) providing an antibody probe according to claim 9 including at least one antibody against an antigen associated with the said pathogen;
(c) probing the pathogen sample with the antibody probe to detect at least one protective antigen; and
(d) isolating the antigen which provides protection for an animal against the pathogen.

14. A method according to any one of claims 1 to 7 or 10 to 13, wherein the sample of the pathogen is selected from parasites, tumors, viruses, chlamydias, rickettsias, mycoplasmas, bacteria, spirochetes, fungi, protozoa, helminths, ectoparasitic arthropods or autoantigens where the antigen is associated with an autoimmune disease.

15. A method according to claim 14, wherein the parasite is selected from Haemonchus contortus, Fasciola hepatica and Taenia hydatigena.

16. A method according to claim 14, wherein the sample of the pathogen is a bacterium or bacterium extract.

17. A method according to claim 16, wherein the bacterium is Corynebacterium pseudotuberculosis.

18. A method according to claim 13, wherein the sample is taken from a disease pathogen at a stage of development during which it is thought to be most susceptible to attack.

19. A method according to claim 18, wherein the disease pathogen is a parasitic cestode infection and the sample is taken from the oncosphere stage.

20. A method according to claim 18, wherein the disease pathogen is a parasitic worm infection and the sample is taken from the larval stage.

21. A method according to claim 18, wherein the disease pathogen is a parasitic fluke infection and the sample is taken from the juvenile fluke stage.

22. A protective antigen prepared by the method according to claim 13 that provides protection for an animal against Taenia hydatigena infections, selected from antigens having a molecular weight of 25 and 34 kilodaltons as determined on SDS-PAGE.

23. A protective antigen prepared by the method according to claim 13 that provides protection for an animal against Haemonchus contortus infections, having a molecular weight of 67 to 75 kilodaltons as determined on SDS-PAGE.

24. A protective antigen prepared by the method according to claim 13 that provides protection for an animal against Fasciola heptica infections, having a molecular weight of 120 to 125 kilodaltons as determined on SDS-PAGE.

25. A protective antigen prepared by the method according to claim 13 that provides protection for an animal against Corynebacterium pseudotuberculosis infections having a molecular weight of 40 kilodaltons as determined on SDS-PAGE.

26. A protective antigen prepared by the method according to claims 13 that provides protection for an animal against Corynebacterium pseudotuberculosis infections, having an NH₂ terminal sequence of Glu, Ser, Ala, Thr, Leu, Ser, Lys, Glu, Pro, Leu, Lys, Ala, Ser, Pro, Gly, Arg, Ala, Asp, Thr, Val, Gly, Val, Gln.

27. A method of preparing a synthetic antigenic polypeptide that provides protection for an animal against a disease pathogen, which method includes:
(a) preparing a cDNA library, or genomic library derived from a sample of a disease pathogen;
(b) providing an antibody probe according to claim 9;
(c) generating synthetic polypeptides from the cDNA library or genomic library;
(d) probing the synthetic polypeptides with the antibody probe; and
(e) isolating the synthetic antigenic polypeptide detected thereby.

28. A method of producing a monoclonal antibody against an antigen of a disease pathogen, which method includes:
(a) providing a B cell capable of producing antibodies against said antigen and obtained from an animal immunized with a protective antigen against the disease pathogen prepared by the method according to claim 13, and a myeloma cell;
(b) fusing the B cell with the myeloma cell;
(c) propagating a hybridoma formed thereby; and
(d) harvesting the antibody produced by said hybridoma.

29. A monoclonal antibody against a protective antigen produced by a method according to claim 28.

30. A vaccine or veterinary composition including a prophylactically effective amount of at least one protective antigen prepared by the method according to claim 13 against a disease pathogen selected from Taenia hydatigena, Haemonchus contortus, Fasciola hepatica and Corynebacterium pseudotuberculosis.

31. A vaccine or veterinary composition including a therapeutically effective amount of at least one monoclonal antibody according to claim 29.

## Patentansprüche

1. Verfahren zur Herstellung von mindestens einem Antikörper gegen einen Krankheitserreger, wobei dieses Verfahren darin besteht, die folgenden Verfahrensschritte zu kombinieren:
(a) Bereitstellen einer biologischen Probe, welche antikörperproduzierende Zellen eines immunen Tieres enthält, welches mit einem Erreger oder Erregerextrakt infiziert oder angegriffen ist, wobei die biologische Probe innerhalb einer geeigneten Periode aus einem Infektionsherd, oder einem Bereich einer Läsion, oder einem Bereich nahe an dem Infektionsherd oder der Läsion oder einem diesen dränierenden Bereich entnommen wurde;
(b) Isolieren der antikörperproduzierenden Zellen aus der biologischen Probe;
(c) Kultivieren der antikörperproduzierenden Zellen aus der biologischen Probe in vitro in einem geeigneten Kulturmedium; und
(d) Ernten des durch die antikörperproduzierenden Zellen produzierten Antikörpers.

2. Verfahren nach Anspruch 1, bei dem die biologische Probe von dem Tier in der Periode entnommen wird, in welcher der Erreger empfindlich ist gegen eine Immunattacke, nachdem das Tier durch den Erreger oder Erregerextrakt infiziert worden ist.

3. verfahren nach Anspruch 1 oder 2, bei dem die von der biologischen Probe isolierten antikörperproduzierende Zellen B-Zellen sind, welche in einer Periode isoliert worden sind, von welcher man annimmt, dass sie eine Sekretions- und/oder eine antikörperproduzierende Periode enthält.

4. Verfahren nach Anspruch 3, bei welchem die B-Zellen innerhalb einer Periode isoliert werden, in welcher der Erreger empfindlich auf eine Immunattacke ist, nachdem das Tier durch den Erreger oder Erregerextrakt infiziert worden ist.

5. Verfahren nach einem der Ansprüche 1 bis 4, bei welchem die antikörperproduzierenden Zellen, die von der Probe isoliert worden sind, Gedächtnis-B-Zellen sind.

6. Verfahren nach einem der Ansprüche 1 bis 5, welches desweitern den Schritte enthält: Hinzufügen eines zellenaktivierenden Mittels zum Kulturmedium, um die kultivierten antikörperproduzierenden Zellen, welche von der biologischen Probe isoliert worden sind, anzuregen und/oder zu vermehren, um Antikörper abzuscheiden und/oder freizusetzen.

7. Verfahren nach Anspruch 6, bei welchem das zellaktivierende Mittel ausgewählt wird aus Mitogenen und Hilfsfaktoren oder ihren synthetischen "-Äquivalenten, aus von Erregern abgeleiteten Molekülen oder einer Kombination derselben.

8. Antikörper, welcher durch das Verfahren nach einem der Ansprüche 1 bis 7 hergestellt wurde.

9. Antikörperprobe mit wenigstens einem Antikörper gegen einen Krankheitserreger, wobei der Antikörper nach einem Verfahren gemäss einem der Ansprüche 1 bis 7 hergestellt worden ist.

10. Verfahren zur Identifizierung von Antigenen, welches besteht aus:
(a) Bereitstellen einen rohen Antigenmischung;
(b) Bereitstellen mindestens eines Antikörpers gegen einen Krankheitserreger, wobei der Antikörper nach einem Verfahren gemäss einem der Ansprüche 1 bis 7 hergestellt worden ist; und
(c) Kupplung der rohen Antigenmischung mit dem Antikörper, um Antigene des Erregers, welcher vom Antikörper gegen den Erreger erkannt worden ist, zu identifizieren.

11. Verfahren zum Läutern eines Antigens, welches Verfahren umfasst:
(a) Bereitstellen einer rohen Antigenmischung;
(b) Bereitstellen eines Antikörpers gegen einen Krankheitserreger, wobei der Antikörper auf einem geeigneten Träger immobilisiert ist und gemäss einem Verfahren nach einem der Ansprüche 1 bis 7 hergestellt worden ist;
(c) Aussetzen der rohen Antigenmischung einer Affinitätschromatographie unter Verwendung des immobilisierten Antikörpers; und
(d) Isolieren des geläuterten Antigens des Erregers, welches von dem immobilisierten Antikörper erkannt worden ist.

12. Verfahren zur Herstellung eines einem Krankheitserreger zugeordneten Antigens, wobei diese Verfahren umfasst:
(a) Bereitstellen einer Probe eines von einem kranken Tier, in einem Entwicklungsstadium entnommenen Erregers, in welchem man annimmt, dass er am empfindlichsten auf einen Angriff ist;
(b) Bereitstellen einer Antikörperprobe gemäss Anspruch 9;
(c) Koppeln der Antigenprobe mit der Antikörperprobe, um wenigstens ein vom Antikörper erkanntes Antigen festzustellen; und
(d) Isolieren des festgestellten Antigens.

13. Verfahren zum Herstellen eines schützenden Antigens, welches einem Tier einen Schutz gegen einen Krankheitserreger verleiht, wobei dieses Verfahren die folgenden Verfahrensschritte umfasst:
(a) Bereitstellen einer Probe des Erregers;
(b) Bereitstellen einer Antikörperprobe gemäss Anspruch 9, welche wenigstens einen Antikörper gegen ein diesem Erreger zugeordnetes Antigen enthält;
(c) Koppeln der Erregerprobe mit der Antikörperprobe, um wenigstens ein schützendes Antigen festzustellen; und
(d) Isolieren des Antigens, welches einem Tier Schutz gegen den Erreger bietet.

14. Verfahren nach einem der Ansprüche 1 bis 7 oder 10 bis 13, bei welchem die Erregerprobe ausgewählt ist aus Parasiten, Tumoren, Viren, Manteltieren, Rickettsien, Mykoplasmen, Bakterien, Spirochätten, Pilzen, Protozoen, Würmern, ektoparasitische Gliederfüssern oder Autoantigenen, bei welchen das Antigen einer autoimmunen Krankheit zugeordnet ist.

15. Verfahren nach Anspruch 14, bei welchem die Parasiten ausgewählt sind von Haemonchus contortus, Fasciola hepatica und Taenia hydatigena.

16. Verfahren nach Anspruch 14, bei welchem die Erregerprobe eine Bakterie oder ein Bakterienextrakt ist.

17. Verfahren nach Anspruch 16, bei welcher die Baktere das Corynebacterium pseudotuberculosis ist.

18. Verfahren nach Anspruch 18, bei welchem die Probe von einem Krankheitserreger in einem Entwicklungsstadium genommen wird, in welchem angenommen wird, dass er am empfindlichsten auf einen Angriff ist.

19. Verfahren nach Anspruch 18, bei welchem der Krankheitserreger eine parasitäre Bandwurminfektion ist und die Probe im onkosphäre Stadium genommen wird.

20. Verfahren nach Anspruch 18, bei welchem der Krankheitserreger eine parasitäre Wurminfektion ist und die Probe im Larvenzustand genommen wird.

21. Verfahren nach Anspruch 18, bei welchem der Krankheitserreger eine parasitäre Saugwurminfektion ist und die Probe im juvenilen Stadium des Saugwurmes genommen wird.

22. Schützendes Antigen, welches durch das Verfahren gemäss Anspruch 13 hergestellt worden ist, welches einem Tier Schutz gegen Taenia hydatigena Infektionen gibt, welches von den Antigenen ausgewählt worden sind, welche ein Molekulargewicht von 25 bis 34 Kilodalton, wie es nach SDS-PAGE bestimmt wird, haben.

23. Schützendes Antigen, welches nach dem Verfahren gemäss Anspruch 13 hergestellt worden ist, welches einem Tier Schutz gegen Haemonchus contortus Infektionen gibt, welche ein Molekulargewicht von 67 bis 75 Kilodalton, wie sie nach SDS-PAGE bestimmt wird, haben.

24. Schützendes Antigen, welches nach dem Verfahren gemäss Anspruch 13 hergestellt worden ist, welches einem Tier Schutz gegen Fasciola heptica Infektionen gibt, welches ein Molekulargewicht von 120 bis 125 Kilodalton, wie es auf SDS-PAGE bestimmt wird, hat.

25. Schützendes Antigen, welches nach dem Verfahren gemäss Anspruch 13 hergestellt worden ist, welches einem Tier Schutz gegen Corynebacterium pseudotuberculosis Infektionen gibt, welches ein Molekulargewicht von 40 Kilodalton, wie es nach SDS-PAGE bestimmt wird, hat.

26. Schützendes Antigen, welches nach dem Verfahren gemäss Anspruch 13 hergestellt worden ist, welches einem Tier Schutz gegen Corynebacterium pseudotuberculosis Infektionen gibt, welches eine NH₂ Endsequenz von Glu, Ser, Ala, Thr, Leu, Ser, Lys, Glu, Pro, Leu, Lys, Ala, ser, Pro, Gly, Arg, Ala, Asp, Thr, Val, Gly, Val, Gln hat.

27. Verfahren zur Herstellung eines synthetischen antigenen Polypeptid, welches einem Tier Schutz gegen einen Krankheitserreger gibt, welches Verfahren umfasst:
(a) Herstellen einer cDNS Bibliothek, oder einer Genomen-Bibliothek, welche aus einer Probe eines Krankheitserregers abgeleitet worden ist;
(b) Bereitstellen einer Antikörperprobe gemäss Anspruch 9;
(c) Erzeugen synthetischer Polypeptide von der cDNS Bibliothek oder Genomen-Bibliothek;
(d) Koppeln den synthetischen Polypeptiden mit der Antikörperprobe; und
(e) Isolieren des synthetischen antigenen Polypeptids, welches dadurch festgestellt wird.

28. Verfahren zur Herstellung eines monoklonalen Antikörpers gegen ein Antigen eines Krankheitserregers, welches Verfahren umfasst:
(a) Bereitstellen einer B-Zelle, welche Antikörper gegen das Antigen produzieren kann und, welches von einem mit einem schützenden Antigen gegen den Krankheitserreger, welches nach dem Verfahren gemäss Anspruch 13 hergestellt worden ist, immunisiert ist, und einer Myelomazelle;
(b) Verschmelzen der B-zelle mit der Myelomazelle;
(c) Vermehren der dadurch gebildeten Hybrodome; und
(d) Ernten der durch dieses Hybrodom erzeugten Antikörpers.

29. Monoklonale Antikörper gegen ein mit dem Verfahren gemäss Anspruch 28 hergestelltes schützendes Antigens.

30. Impfstoff oder veterinäre Zusammensetzung mit einer prophylaktisch wirksamen Menge von wenigstens einem schützenden Antigen, welches durch das Verfahren gemäss dem Anspruch 13 hergestellt worden ist, gegen einen Krankheitserreger, welcher aus Taenia hydatigena, Haemonchus contortus, Fasciola hepatica und Corynebacterium pseudotuberculosis ausgewählt ist.

31. Impfstoff oder veterinäre Zusammensetzung mit einer therapeutisch wirksamen Menge von wenigstens einem monoklonalen Antikörper nach Anspruch 29.

## Revendications

1. Procédé pour la préparation d'au moins un anticorps contre un agent pathogène, ladite méthode comprenant la combinaison des étapes suivantes:
(a) fournir un échantillon biologique contenant des cellules produisant des anticorps provenant d'un animal immunisé infecté avec, ou attaqué par, un agent pathogène ou un extrait d'agent pathogène, ledit échantillon biologique étant pris pendant une période appropriée d'un lieu d'infection, ou d'une zone d'une lésion, ou d'une zone proche de ou drainant le lieu d'infection ou de la lésion;
(b) isoler les cellules produisant des anticorps de l'échantillon biologique;
(c) cultiver les cellules produisant des anticorps de l'échantillon biologique in vitro dans un milieu de culture approprié; et
(d) recueillir l'anticorps produit par les cellules produisant des anticorps.

2. Procédé selon la revendication 1, dans lequel l'échantillon biologique est pris sur un animal pendant la période où l'agent pathogène est vulnérable à l'attaque immunisante après que l'animal a été infecté par l'agent pathogène ou l'extrait d'agent pathogène.

3. Procédé selon la revendication 1 ou 2, dans lequel les cellules produisant des anticorps isolés de l'échantillon biologique sont des cellules B isolées pendant une période connue pour inclure une période de sécrétion et/ou de production d'anticorps.

4. Procédé selon la revendication 3, dans lequel les cellules B sont isolées pendant une période où l'agent pathogène est vulnérable à l'attaque immunisante après que l'animal a été infecté par l'agent pathogène ou l'extrait d'agent pathogène.

5. Procédé selon une quelconque des revendications 1 à 4, caractérisé en ce que les cellules produisant des anticorps isolés de l'échantillon biologique sont des cellules B à mémoire.

6. Procédé selon une quelconque des revendications 1 à 5, comprenant en outre l'étape d'ajouter un agent activant les cellules au milieu de culture pour activer et/ou proliférer les cellules cultivées produisant des anticorps isolés de l'échantillon biologique pour sécréter et/ou libérer des anticorps.

7. Procédé selon la revendication 6, dans lequel l'agent activateur des cellules est choisi des mitogènes et des facteurs auxiliaires (helper) ou de leurs équivalents synthétiques, des molécules dérivées d'agents pathogènes de molécules ou une combinaison de ceux-ci.

8. Anticorps préparé par le procédé selon l'une quelconque des revendications 1 à 7.

9. Echantillon d'anticorps comprenant au moins un anticorps contre un agent pathogène, ledit anticorps ayant été produit par un procédé selon une quelconque des revendications 1 à 7.

10. Procédé pour identifier des antigènes comprenant:
(a) prévoir un mélange antigène cru;
(b) prévoir au moins un anticorps contre un agent pathogène, ledit anticorps ayant été produit par un procédé selon une quelconque des revendications 1 à 7; et
(c) coupler le mélange antigène cru avec l'anticorps pour identifier des antigènes de l'agent pathogène reconnus par l'anticorps contre l'agent pathogène.

11. Procédé pour purifier un antigène, comprenant:
(a) prévoir un mélange antigène cru;
(b) prévoir un anticorps contre un agent pathogène, ledit anticorps étant immobilisé sur un support approprié et ayant été produit par un procédé selon une quelconque des revendications 1 à 7;
(c) soumettre le mélange antigène cru à la chromatographie d'affinité en utilisant l'anticorps immobilisé; et
(d) isoler l'antigène purifié de l'agent pathogène reconnu par l'anticorps immobilisé.

12. Procédé pour préparer un antigène associé à un agent pathogène, comprenant:
(a) prévoir un échantillon d'un agent pathogène pris d'un animal malade dans un état de développement pendant lequel il est admis que l'agent pathogène est le plus susceptible à l'attaque;
(b) prévoir un échantillon d'anticorps selon la revendication 9;
(c) coupler l'échantillon pathogène avec l'échantillon anticorps pour détecter au moins un antigène reconnu par l'anticorps; et
(d) isoler l'antigène détecté.

13. Procédé de préparation d'un antigène protecteur qui fournit à un animal une protection contre un agent pathogène, ladite méthode comprenant les étapes de:
(a) prévoir un échantillon d'agent pathogène;
(b) prévoir un agent d'anticorps selon la revendication 9 comprenant au moins an anticorps contre un anticorps associé audit agent pathogène;
(c) coupler l'échantillon d'agent pathogène avec l'échantillon d'anticorps pour détecter au moins un antigène protecteur; et
(d) isoler l'antigène qui fournit à un animal la protection contre l'agent pathogène.

14. Procédé selon une quelconque des revendications 1 à 7 et 10 à 13, dans lequel l'échantillon de l'agent pathogène est choisi des parasites, tumeurs, virus, chlamydia, rickettsias, mycoplasmes, bactéries, spirochètes, champignons, protozoaires, helminthes, arthropodes ectoparasites ou auto-antigènes où l'antigène est associé à une maladie autoimmunisante.

15. Procédé selon la revendication 14, dans lequel le parasite est choisi de Haemonchus contortus, Fasciola hepatica et Taenia hydatigena.

16. Procédé selon la revendication 14, dans lequel l'échantillon d'agent pathogène est une bactérie ou un extrait de bactérie.

17. Procédé selon la revendication 16, dans lequel la bactérie est Corynebacterium pseudotuberculosis.

18. Procédé selon la revendication 13, dans lequel l'échantillon est pris d'un agent pathogène à un état de développement dans lequel on pense qu'il est le plus susceptible à l'attaque.

19. Procédé selon la revendication 10, dans lequel l'agent pathogène est une infection cestoïde parasitaire et l'échantillon est pris au stage d'oncosphère.

20. Procédé selon la revendication 19, dans lequel l'agent pathogène est une infection de ver parasitaire et l'échantillon est pris à l'état de larve.

21. Procédé selon la revendication 18, dans lequel l'agent pathogène est une infection parasitaire de trématode et l'échantillon est pris à l'état juvénile du trématode.

22. Antigène protecteur préparé par le procédé selon la revendication 13, qui fournit à un animal une protection contre des infections de Taenia hydatigena, choisi des antigènes ayant un poids moléculaire de 25 à 34 kilodaltons, tel que déterminé sur SDS-PAGE.

23. Antigène protecteur préparé par le procédé selon la revendication 13, qui fournit à un animal une protection contre des infections de Haemonchus contortus, ayant un poids moléculaire de 67 à 75 kilodaltons, tel que déterminé sur SDS-PAGE.

24. Antigène protecteur préparé par le procédé selon la revendication 13, qui fournit à un animal une protection contre des infections de Fasciola heptica, ayant un poids moléculaire de 120 à 125 kilodaltons, tel que déterminé sur SDS-PAGE.

25. Antigène protecteur préparé par le procédé selon la revendication 13, qui fournit à un animal une protection contre des infections de Corynebacterium pseudotuberculosis ayant un poids moléculaire de 40 kilodaltons, tel que déterminé sur SDS-PAGE.

26. Antigène protecteur préparé par le procédé selon la revendication 13, qui fournit à un animal une protection contre des infections Corynebacterium pseudotuberculosis, ayant une séquence terminale NH₂ de Glu, Ser, Ala, Thr, Leu, Ser, Lys, Glu, Pro, Leu, Lys, Ala, Ser, Pro, Gly, Arg, Ala, Asp, Thr, Val, Gly, Val, Gln.

27. Procédé pour préparer un polypeptide antigénique synthétique qui fournit à un animal une protection contre un agent pathogène, ledit procédé comprenant:
(a) préparer une bibliothèque de ADNc, ou une bibliothèque génomique dérivée d'un échantillon d'un agent pathogène;
(b) prévoir un échantillon d'anticorps selon la revendication 9;
(c) produire des polypeptides synthétiques à partir de la bibliothèque ADNc ou de la bibliothèque génomique;
(d) coupler les polypeptides synthétiques avec l'échantillon d'anticorps; et
(e) isoler le polypeptide antigénique synthétique ainsi détecté.

28. Procédé pour produire un anticorps monoclonal contre un antigène d'un agent pathogène, ladite méthode comprenant:
(a) prévoir une cellule B capable de produire des anticorps contre ledit antigène et obtenir d'un animal immunisé avec un antigène protecteur contre l'agent pathogène et préparé par le procédé selon la revendication 13 et une cellule de myélome;
(b) fusionner la cellule B avec la cellule de myélome;
(c) propager un hybridome ainsi formé; et
(d) recueillir l'anticorps produit par ledit hybridome.

29. Anticorps monoclonal contre un agent protecteur produit par un procédé selon la revendication 28.

30. Vaccin ou composition vétérinaire comprenant une quantité efficace prophylactique d'au moins un antigène protecteur préparé par le procédé selon la revendication 13 contre un agent pathogène choisi de Taenia hydatigena, Haemonchus contortus, Fasciola hepatica et Corynebacterium pseudotuberculosis.

31. Vaccin ou composition vétérinaire comprenant une quantité efficace thérapeutique d'au moins un anticorps monoclonal selon la revendication 29.
